# FASCICULE DE BREVET EUROPEEN

(11) **EP 1 276 509 B1**
(45) Date de publication et mention de la délivrance du brevet: **18.06.2008**
(21) Numéro de dépôt: 01928034.6
(22) Date de dépôt: 23.04.2001
(51) Int. Cl.: A61L 2/26, A61L 2/24, A61B 19/02

(54) **ENSEMBLE DE STERILISATION D'INSTRUMENTS OU D'APPAREILS MEDICAUX**
VORRICHTUNG ZUR STERILISATION VON MEDIZINISCHEN INSTRUMENTEN ODER GERÄTEN
SET FOR STERILISING MEDICAL INSTRUMENTS OR APPLIANCES

(30) Priorité: 25.04.2000 FR 0005229
(43) Date de publication de la demande: 22.01.2003
(73) Titulaire: Metrolog, Services Metrologiques, 31670 Labege Cedex (FR)
(72) Inventeur: LACABANNE, Jean-Paul, F-31130 Balma (FR)
(74) Mandataire: Cabinet BARRE LAFORGUE & associés
(86) Numéro de dépôt international: PCT/FR2001/001237
(87) Numéro de publication internationale: WO 2001/080908

(56) Documents cités:
- EP-A- 0 630 820
- DE-A- 3 632 674
- US-A- 4 948 566

## Description

L'invention concerne un ensemble de stérilisation, notamment d'instruments ou d'appareils médicaux.

Dans les centres de soins, les laboratoires pharmaceutiques ou autres établissements qui sont amenés à assurer la stérilisation d'appareils ou d'instruments, on utilise des conteneurs métalliques dont le but est de contenir notamment des instruments chirurgicaux pendant la phase de stérilisation, et de conserver ensuite leur état stérile.

A ce jour, ces conteneurs sont de deux types principaux :
- conteneur à filtre : un filtre en papier spécifique permet le passage de la vapeur d'eau, mais ne permet pas le passage des germes qui pourraient contaminer le produit,
- conteneur à soupapes : un système de soupapes permet le passage de la vapeur et son extraction et se ferme pour assurer l'étanchéité du conteneur.

Dans la pratique, ces deux types de conteneurs ne donnent toutefois pas entière satisfaction. En effet, les filtres en papier peuvent être endommagés et donc ne plus assurer d"'étanchéité" vis-à-vis des germes. Les soupapes n'assurent quant à elles pas toujours une étanchéité correcte.

En vue de pallier ces inconvénients dont les conséquences peuvent être très néfastes à la santé des patients, deux autres solutions ont été actuellement proposées.

La première de ces solutions décrite dans le brevet US-4.948.566 consiste à proposer une unité de stérilisation comportant une centrale de contrôle des paramètres de stérilisation, et une pluralité de verrous répartis dans la chambre de stérilisation, adaptés pour engendrer l'ouverture et la fermeture de conteneurs disposés dans cette dernière.

Bien que cette solution semble satisfaisante du point de vue de la qualité de la stérilisation, elle présente toutefois l'inconvénient de requérir des unités de stérilisation très spécifiques et des conteneurs dédiés à ces unités de stérilisation. Par conséquent, en effet, elle impose de renouveler l'intégralité des unités de stérilisation ainsi que l'intégralité des conteneurs, et elle s'avère d'un coût de revient très élevé.

La seconde solution décrite dans le brevet DE-3.632.674 consiste à proposer un conteneur comportant, incorporés dans le couvercle dudit conteneur, des moyens électroniques de contrôle d'au moins un paramètre de stérilisation, au moins un accumulateur d'énergie, une unité de commandé asservie aux moyens de contrôle et adaptée, à réception d'un signal émis par ces moyens de contrôle, pour libérer l'énergie des accumulateurs et entraîner la fermeture du couvercle.

Bien que de tels conteneurs semblent également satisfaisants du point de vue de la qualité de la stérilisation, ils présentent toutefois un inconvénient majeur. En effet, selon cette solution, tous les moyens de gestion et de contrôle sont incorporés lors de la fabrication dans le couvercle de chaque conteneur, et lesdits conteneurs présentent par conséquent un prix de revient très élevé. De plus, la réalisation technique de tels conteneurs s'avère très délicate.

Par ailleurs, parallèlement à ces solutions, et dans le but d'assurer une stérilisation de qualité et de pouvoir la contrôler pour une traçabilité totale des dispositifs médicaux depuis la décontamination jusqu'à la réutilisation, il est nécessaire de vérifier que les paramètres de stérilisation ont bien été atteints. A cet effet, jusqu'à un passé récent, l'utilisation d'intégrateurs papiers et les analyses biochimiques jouaient ce rôle. Depuis quelques années, des enregistreurs électroniques autonomes de pression et de température permettent de contrôler, par lecture des données enregistrées dans leur mémoire interne, que les paramètres de stérilisation ont été atteints et donc de valider le cycle en question. Cette méthode est parfois appelée "libération paramétrique des charges". Des appareils de ce type sont d'ailleurs produits par la demanderesse sous les marques déposées "STERILOG" et "MICROLOG".

La présente invention vise à utiliser cette technologie et se propose de fournir un ensemble de stérilisation incorporant un conteneur et un enregistreur électronique, d'une part dont le prix de revient est sensiblement équivalent à celui des conteneurs et enregistreurs actuels, et d'autre part garantissant que les paramètres de stérilisation ont été respectés.

A cet effet, l'invention vise un ensemble de stérilisation, notamment d'instruments ou d'appareils médicaux, comportant un conteneur comprenant un récipient et des moyens d'obturation dudit récipient aptes à l'obturer de façon étanche, et des moyens électroniques d'enregistrement d'au moins un paramètre de stérilisation lors des phases de stérilisation.

Selon l'invention, cet ensemble de stérilisation se caractérise en ce que :
- le conteneur comprend :
   - une soupape disposée à l'intérieur du conteneur en regard d'une paroi, dite paroi de stérilisation, dudit conteneur agencée pour permettre la pénétration dans ce dernier d'un agent stérilisateur,
   - un siège d'appui de la soupape ménagé à l'intérieur du conteneur de façon que ladite soupape assure l'obturation du récipient, dans une position dite de fermeture,
   - des moyens d'étanchéité aptes à assurer une étanchéité entre la soupape et son siège d'appui dans la position de fermeture de ladite soupape,
   - des moyens élastiques disposés entre la paroi de stérilisation et la soupape de façon à solliciter ladite soupape vers sa position de fermeture du récipient,
   - et des moyens d'accrochage réalisés d'un seul tenant avec la soupape et agencés pour s'étendre à l'extérieur du conteneur au travers d'un orifice ménagé dans la paroi de stérilisation, lors d'un effort exercé sur ladite soupape à l'encontre des moyens élastiques, adapté pour amener cette dernière vers une position dite d'ouverture du récipient.
- les moyens d'enregistrement électronique comprennent un appareil électronique autonome adapté pour être accolé à la paroi de stérilisation du conteneur, ledit appareil électronique :

- comprenant des moyens de verrouillage adaptés pour coopérer avec les organes d'accrochage de la soupape, de façon à maintenir cette dernière dans sa position d'ouverture,
- comprenant des moyens de déclenchement automatique des moyens de verrouillage aptes à engendrer la libération des organes d'accrochage de la soupape et à autoriser le déplacement de cette dernière vers sa position de fermeture sous l'action des moyens élastiques,
- étant programmé pour activer les moyens de déclenchement lorsque chaque paramètre de stérilisation est respecté.

Selon l'invention, la gestion de la fermeture de la soupape en fonction de paramètres de stérilisation, est obtenue grâce aux moyens d'accrochage et de verrouillage originaux dont sont dotés ladite soupape et l'appareil. électronique autonome d'enregistrement.

Cette gestion est donc obtenue moyennant l'utilisation de conteneurs et d'appareils électroniques d'un prix de revient sensiblement équivalent à celui des conteneurs et appareils électroniques actuels. De plus, l'appareil électronique, qui constitue l'élément dont le coût de revient est notablement le plus élevé, étant séparable du conteneur après stérilisation, le nombre requis de tels appareils électroniques dans un établissement est très inférieur à celui des conteneurs, et au plus égal à la capacité des stérilisateurs.

Au contraire du dispositif décrit dans le brevet DE-3.632.674, l'invention a ainsi consisté à fournir une solution très originale qui permet de dissocier automatiquement l'appareil électronique du conteneur après stérilisation, d'où un coût de revient des conteneurs comparable à celui des conteneurs classiques actuels, et un nombre d'appareils électroniques requis très inférieur à celui des conteneurs, et comparable à celui requis actuellement.

De plus, il convient de souligner que l'ensemble de stérilisation selon l'invention permet de garantir que les paramètres de stérilisation ont été atteints avant de fermer de façon sure et contrôlée le conteneur de stérilisation.

Selon un mode de réalisation avantageux de l'invention :
- la paroi de stérilisation est constituée d'un couvercle, dit sur-courvercle, de forme adaptée pour coiffer le récipient et doté de moyens de solidarisation audit récipient,
- le siège d'appui de la soupape est constitué d'un couvercle de forme adaptée pour recouvrir le récipient, et percé d'au moins un orifice de passage d'un agent stérilisateur, apte à être obturé par la dite soupape dans la position de fermeture de cette dernière,
- des moyens d'étanchéité sont disposés entre le récipient et le couvercle de façon à assurer l'étanchéité du conteneur dans la position de fermeture de la soupape.

Selon ce mode de réalisation, le conteneur comporte donc un sur-couvercle qui sert de support à l'appareil électronique et qui permet de comprimer les moyens élastiques lors du déplacement de la soupape vers sa position d'ouverture, et un couvercle qui sert de siège à la soupape et assure l'étanchéité du conteneur dans la position de fermeture de ladite soupape. Selon ce mode de réalisation, deux variantes de réalisation avantageuses peuvent, en outre, être envisagées :
- Selon la première variante le couvercle et le sur-couvercle sont réalisés d'un seul tenant de façon à former un ensemble séparable du récipient et incorporant la soupape,
- Selon la seconde variante, le couvercle et le sur-couvercle consistent en deux éléments distincts, la soupape étant disposée sous le sur-couvercle et reliée à ce dernier par les moyens élastiques.

Par ailleurs, de façon avantageuse, selon l'invention, le couvercle est percé d'un orifice central, la soupape étant constituée d'une plaque dotée en sous-face d'un doigt de guidage apte à coulisser dans ledit orifice. Ce guidage de la soupape permet, en effet, de garantir la parfaite coïncidence des moyens d'étanchéité disposés entre ladite soupape et le couvercle.

De plus, concernant ces moyens d'étanchéité, le couvercle comporte avantageusement une portion centrale percée d'une pluralité d'orifices de passage d'un agent stérilisateur, la soupape étant constituée d'une plaque adaptée pour recouvrir ladite portion centrale, et lesdits couvercle et soupape comportant des moyens d'étanchéité ménagés de façon à être situés sur le pourtour de cette portion centrale.

Par ailleurs, le sur-couvercle comporte avantageusement une zone centrale en renfoncement de dimensions adaptées pour loger l'appareil électronique. Un tel renfoncement permet, en effet, d'empiler les conteneurs munis de leur appareil électronique en vue notamment de leur stockage.

De plus, en vue de faciliter la pénétration de l'agent stérilisateur, le sur-couvercle est avantageusement percé d'au moins un orifice.

Selon un autre mode de réalisation avantageux, les moyens d'accrochage de la soupape et les moyens élastiques sollicitant ladite soupape vers sa position de fermeture du récipient, sont adaptés pour que lesdits moyens d'accrochage ne fassent pas saillie à l'extérieur de la paroi de stérilisation, dans la position de fermeture de la soupape. Cette disposition permet de préserver ces moyens d'accrochage contre tout risque d'endommagement, une fois l'appareil électronique séparé du conteneur.

Selon un autre mode de réalisation avantageux de l'invention :
- les moyens d'accrochage de la soupape et les moyens de verrouillage de l'appareil électronique comportent deux couronnes de formes adaptées pour venir s'emboîter l'une dans l'autre, et dotées d'organes de blocage relatif en translation axiale, une desdites couronnes étant composée de doigts flexibles radialement,
- les moyens de verrouillage de l'appareil électronique comportent un verrou disposé de façon à être repoussé par les organes d'accrochage de la soupape lors du déplacement de cette dernière vers sa position d'ouverture, et sollicité par des moyens élastiques aptes à le maintenir dans une position de butée radiale des doigts flexibles.
- les moyens de déclenchement automatique sont adaptés pour entraîner un déplacement du verrou, à l'encontre de l'action des moyens élastiques, vers une position où il libère les doigts flexibles.

Dans ce cas, en outre, et de façon avantageuse, selon l'invention :
- les moyens d'accrochage de la soupape consistent en la couronne composée de doigts flexibles, ladite couronne étant de forme adaptée pour venir coulisser à l'extérieur de la couronne des moyens de verrouillage de l'appareil électronique,
- le verrou de l'appareil électronique consiste en une pièce annulaire de forme adaptée pour venir enserrer les doigts flexibles.

De plus, et de façon avantageuse, selon l'invention:
- la couronne des moyens de verrouillage de l'appareil électronique comporte un tronçon d'extrémité présentant une face externe conique formant une rampe d'insertion à l'intérieur de la couronne de la soupape,
- les organes de blocage en translation des couronnes consistent en un bossage annulaire et une gorge annulaire de formes conjuguées, l'organe de blocage ménagé sur les doigts flexibles étant situé au niveau de l'extrémité de ces derniers,
- le verrou est réalisé en un matériau magnétique, les moyens de déclenchement dudit verrou consistant en un électro-aimant.

D'autres caractéristiques, buts et avantages de l'invention ressortiront de la description détaillée qui suit en référence aux dessins annexés qui en représentent à titre d'exemples non limitatifs, deux modes de réalisation préférentiels.

Sur ces dessins :
- la figure 1 est une vue en perspective d'un premier mode de réalisation d'un ensemble de stérilisation conforme à l'invention,
- la figure 2 est une coupe axiale par un plan vertical A de cet ensemble de stérilisation, dans la position ouverte de la soupape,
- la figure 3 en est une coupe axiale par le plan A, dans la position fermée de la soupape,
- la figure 4 est une coupe partielle par le plan A, à échelle agrandie, de détail des moyens d'accrochage et de verrouillage,
- et la figure 5 est une coupe axiale par le plan A représentant une variante de réalisation d'un ensemble de stérilisation conforme à l'invention.

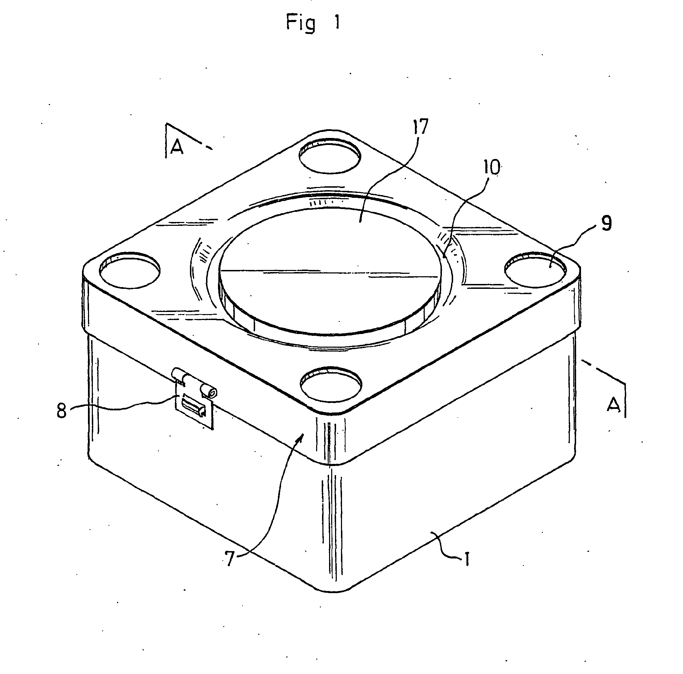

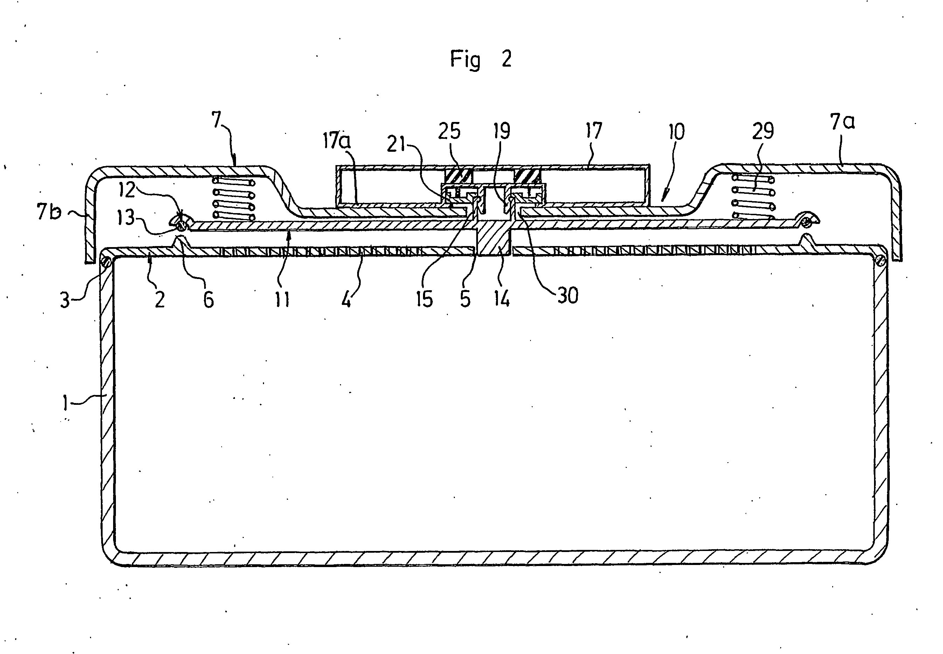

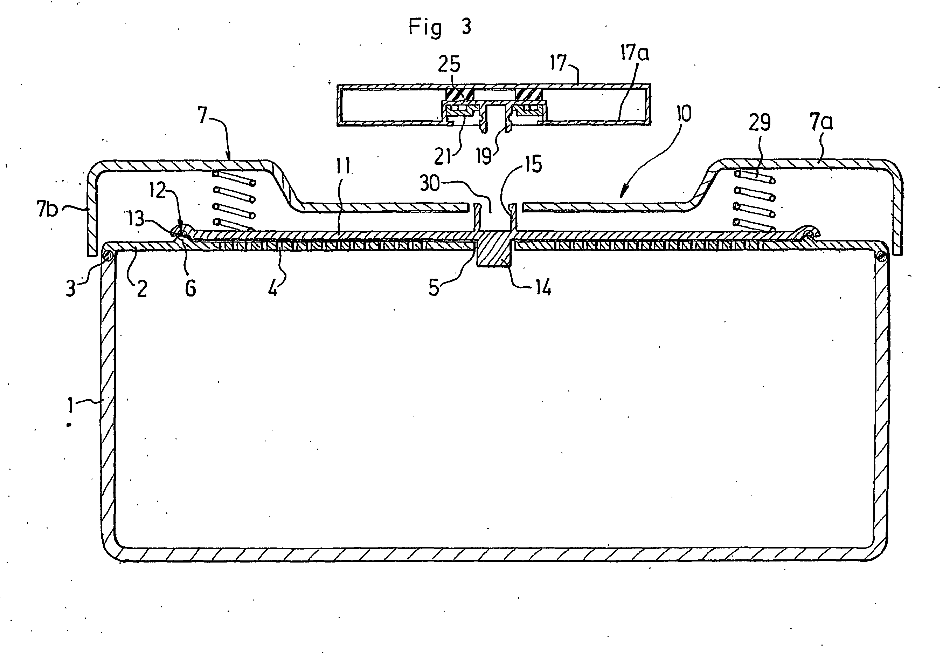

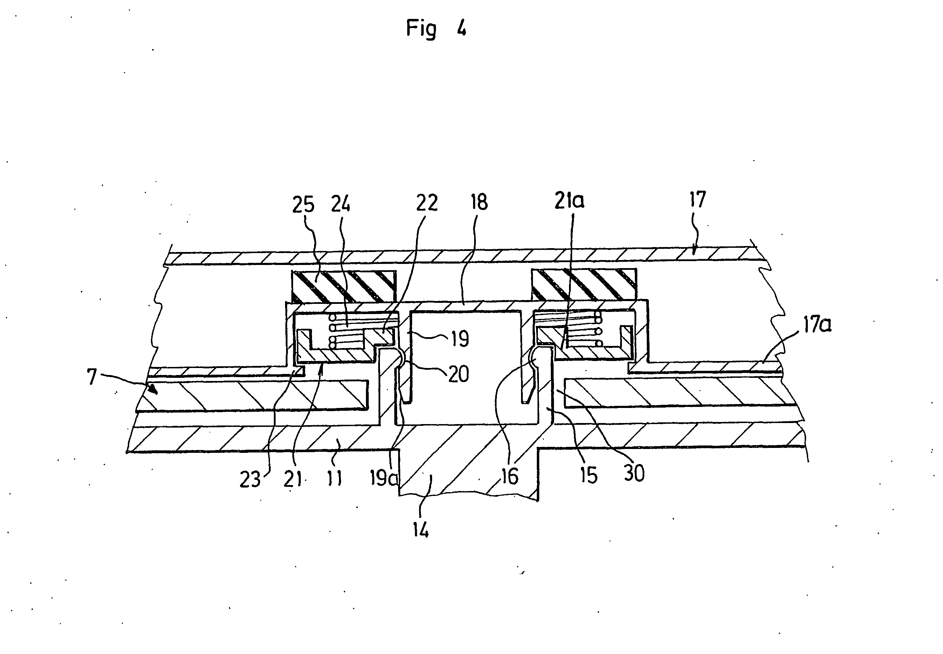

L'ensemble de stérilisation représenté aux figures 1 à 4, comprend, en premier lieu, un récipient métallique 1 de forme classique parallélépipédique rectangle sur lequel vient reposer un couvercle 2 avec interposition, entre lesdits récipient et couvercle, d'un joint d'étanchéité périphérique 3.

Le couvercle 2 comporte, en outre, une portion de surface centrale circulaire percée d'une pluralité de petits orifices tels que 4 répartis sur ladite portion de surface. Il est également percé d'un orifice axial 5 de plus grand diamètre.

Le couvercle 2 comporte, enfin, en saillie par rapport à sa face supérieure, une lèvre circulaire 6 ménagée sur le pourtour de la portion de surface centrale percée d'orifices 4.

L'ensemble de stérilisation comporte, en outre, un sur-couvercle 7 comportant une paroi supérieure 7a et un rebord périphérique 7b sur lequel sont disposés des moyens classiques 8 de verrouillage dudit sur-couvercle sur le récipient 1, dans une position où un volume est ménagé entre la paroi supérieure 7a de ce sur-couvercle 7 et le couvercle 2.

La paroi supérieure 7a de ce sur-couvercle 7 est percée d'orifices tels que 9, en l'exemple au nombre de quatre ménagés dans chacun des angles de cette paroi supérieure, destinés au passage d'un agent stérilisateur.

Cette paroi 7a présente, en outre, un renfoncement 10 dans sa portion centrale, définissant un logement de forme générale cylindrique apte à loger l'appareil électronique décrit plus loin.

L'ensemble de stérilisation comporte, par ailleurs, une soupape 11 constituée d'une plaque de dimensions conjuguées de la surface délimitée par la lèvre 6 du couvercle 2, adaptée pour venir recouvrir cette surface, ladite soupape étant dotée d'une gorge périphérique 12 logeant un joint d'étanchéité 13 disposé de façon à coopérer avec ladite lèvre 6, de façon à assurer une fermeture étanche du récipient 1.

En vue de garantir une parfaite coïncidence entre la lèvre 6 et le joint 13, la soupape 11 comprend, en outre, en saillie par rapport à sa sous-face, un doigt axial de guidage 14 apte à se déplacer dans l'orifice axial 5 du couvercle 2.

Cette soupape 11 comporte, enfin, des moyens d'accrochage axiaux adaptés pour :
- venir en saillie dans le renfoncement 10 du sur-couvercle 7 au travers d'un orifice axial 30 ménagé dans la paroi supérieure 7a dudit sur-couvercle, dans une position d'ouverture du récipient 1 où ladite soupape est décollée du couvercle 2,
- être situés en retrait ou au plus affleurer la paroi supérieure 7a du sur-couvercle 7, dans la position de fermeture du récipient 1, où la soupape repose de façon étanche sur le couvercle 2.

Ces moyens d'accrochage consistent en une couronne axiale 15, en saillie par rapport à la face supérieure de la soupape 11, constituée de doigts flexibles aptes à pouvoir s'ouvrir radialement, et dotés d'un bossage interne 16 vers leur extrémité supérieure.

En vue d'assurer le placage de la soupape 11 sur le couvercle 2, dans la position de fermeture du récipient 1, l'ensemble de stérilisation comporte, en outre, des ressorts tels que 29 (ou tout moyen élastique équivalent tel que lame ...) disposés entre ladite soupape et la paroi supérieure 7a du sur-couvercle 7, dans des zones situées en dehors de l'empreinte des orifices 9 et du renfoncement 10.

L'ensemble de stérilisation selon l'invention comprend, enfin, un appareil électronique autonome d'enregistrement des paramètres de stérilisation tels que température et pression, adapté, en outre, pour verrouiller la soupape 11 dans sa position d'ouverture du récipient 1, durant la stérilisation, et pour libérer cette soupape 11 et permettre son déplacement vers sa position de fermeture du récipient 1, sous l'action des ressorts 29, une fois les paramètres de stérilisation respectés.

Cet appareil électronique 17 comporte un boîtier étanche réalisé en un matériau amagnétique, de forme générale cylindrique et de dimensions adaptées pour venir se loger dans le renfoncement 10 du sur-couvercle 7.

Ce boîtier 17 intègre, de façon classique, l'électronique et la source de puissance (piles ou batteries). Il comporte, en outre, un renfoncement axial de forme cylindrique ménagé dans la face inférieure 17a dudit boîtier et destiné à loger les moyens de verrouillage de la soupape 11.

Ces moyens de verrouillage comprennent, en premier lieu, une couronne rigide 19 s'étendant axialement à partir du fond du renfoncement 18, et de longueur adaptée pour faire saillie en sous-face du boîtier 17. Cette couronne 19 de diamètre adapté pour venir s'insérer entre les doigts flexibles 15 de la soupape 11, comporte une gorge annulaire externe 20 ménagée de façon à venir loger les bossages 16 desdits doigts flexibles, dans la position d'ouverture du récipient 1 de ladite soupape.

Cette couronne 19 comporte, en outre, un tronçon d'extrémité inférieur 19a présentant une face externe conique formant une rampe d'insertion entre les doigts flexibles 15.

Les moyens de verrouillage comportent, en outre, un verrou 21 réalisé en un matériau magnétique et destiné à verrouiller les doigts flexibles 15 dans la position où le bossage 16 de ces derniers est logé dans la gorge 20 de la couronne 19.

A cet effet, ce verrou 21 se compose d'une pièce annulaire disposée dans le renfoncement 18 du boîtier 17, dans un volume annulaire périphérique dudit renfoncement de largeur correspondant à la distance entre la paroi périphérique de ce dernier et la face externe des doigts flexibles 15 lorsque le bossage 16 de ces derniers est logé dans la gorge 20 de la couronne 19.

Le corps de cette pièce annulaire 21 présente une section en forme de U adapté pour qu'une des ailes 21 a dudit U serve de butée radiale de verrouillage des doigts flexibles 15. Cette aile 21 a est, en outre, prolongée par un rebord externe 22 perpendiculaire à ladite aile.

Les moyens de verrouillage comprennent, par ailleurs, un ressort 24 disposé dans le renfoncement 18, entre le fond de ce dernier et le verrou 21 et adapté pour solliciter ce dernier vers sa position de verrouillage des doigts flexibles. Enfin, un rebord périphérique 23 coplanaire avec la paroi inférieure 17a du boîtier 17 fait saillie à l'intérieur du renfoncement 18 de façon à servir de butée au verrou 21 sollicité par le ressort 24.

En dernier lieu, l'appareil électronique 17 comprend des moyens de déclenchement automatique du verrou 21 aptes à engendrer la libération des doigts flexibles 15, et consistant en un électro-aimant 25 formé d'une bobine plate disposée dans le boîtier 17a à l'aplomb dudit verrou de façon à pouvoir attirer ce dernier, sur commande, à l'encontre de l'action du ressort 24.

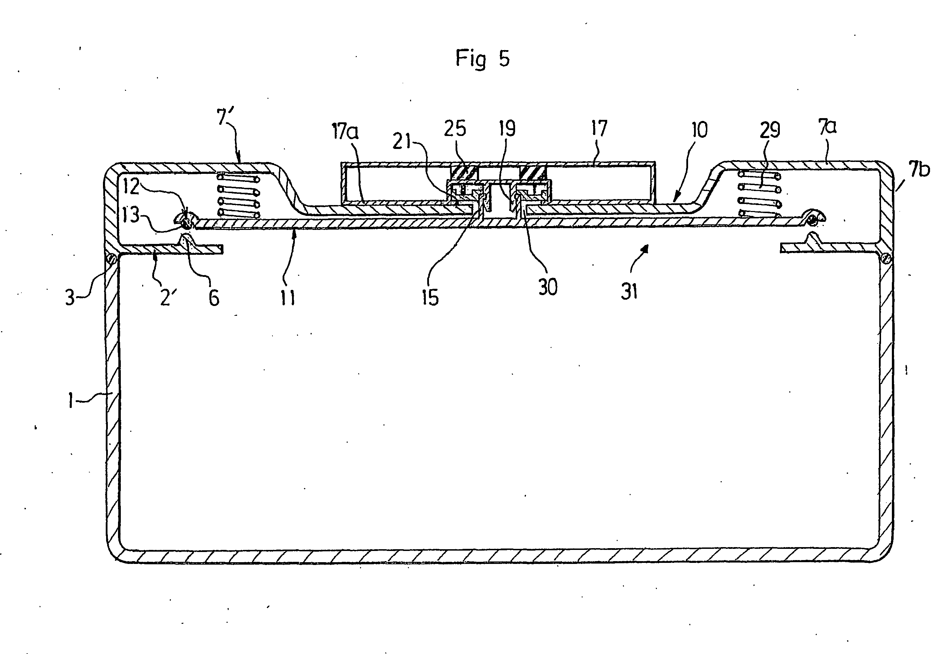

La variante de réalisation de l'ensemble de stérilisation conforme à l'invention représenté à la figure 5 est, dans son ensemble, similaire à celle décrite ci-dessus, et les mêmes références ont été utilisées sur cette figure pour désigner les éléments identiques des deux modes de réalisation.

La seule différence réside dans le fait que, selon ce mode de réalisation, le couvercle 2' et le sur-couvercle 7' sont réalisés d'un seul tenant.

Selon ce mode de réalisation, le sur-couvercle 7' présente une forme identique à celui précédemment décrit, tandis que le couvercle 2' consiste en une couronne interne solidaire de la base du rebord périphérique 7b dudit sur-couvercle, sur laquelle est ménagée la lèvre d'étanchéité 6, et délimitant un orifice central 31 apte à être obturé par la soupape 11.

Le fonctionnement des ensembles de stérilisation ci-dessus décrits est le suivant :

En vue de l'armement, l'appareil électronique 17 est placé sur le sur-couvercle 7 dans son logement 10. A ce moment, la soupape 11 est normalement fermée. Les ressorts 29 de la soupape 11 sont alors comprimés manuellement de manière à ce que les doigts 15 entrent dans l'appareil électronique 17, soient forcés de par leur flexibilité sur la couronne 19 et repoussent le verrou 21 en comprimant le ressort 24. Lorsque le bossage 16 des doigts 15 se trouve en face de la gorge 2 de la couronne 19, et grâce à l'élasticité desdits doigts, ce bossage 16 pénètre dans cette gorge 10 libérant de ce fait un espace suffisant pour que le verrou 21 soit repoussé vers sa position de verrouillage par le ressort 24. Le système est alors armé, la soupape 11 est maintenue ouverte et l'appareil électronique 17 est verrouillé sur le sur-couvercle 7 du conteneur. Le sur-couvercle 7 peut maintenant être posé sur le récipient 1 du conteneur muni du couvercle 2, et verrouillé sur ce récipient 1.

Pendant le cycle de stérilisation, l'appareil électronique 17 mesure et enregistre les paramètres physiques (i.e. pression et température). Son logiciel interne compare les données enregistrées à des critères programmés. Lorsque ces critères ont été satisfaits, l'appareil électronique 17 envoie un signal de puissance à l'électro-aimant 25 qui se trouve activé. Le champ magnétique qui en résulte attire le verrou 21 en comprimant le ressort 24, libérant ainsi les doigts 15. La force des ressorts 29 tend à repousser la soupape 11 et à faire échapper le bossage 16 des doigts 15 de la gorge 20. La soupape 11 se referme et l'appareil électronique 17 est libre.

L'application industrielle de cette invention est la stérilisation d'appareils ou d'instruments médico-chirurgicaux dans des conteneurs étanches avec l'assurance que l'état stérile a été obtenu (par libération paramétrique) et sera conservé.

## Revendications

1. - Ensemble de stérilisation, notamment d'instruments ou d'appareils médicaux, comportant un conteneur comprenant un récipient (1) et des moyens d'obturation (2, 7, 11 ; 2, 7', 11) dudit récipient aptes à l'obturer de façon étanche, et des moyens électroniques (17) d'enregistrement d'au moins un paramètre de stérilisation lors des phases de stérilisation, ledit ensemble de stérilisation étant **caractérisé en ce que** :
- le conteneur comprend :
• une soupape (11) disposée à l'intérieur du conteneur en regard d'une paroi (7 ; 7'), dite paroi de stérilisation, dudit conteneur agencée pour permettre la pénétration dans ce dernier d'un agent stérilisateur,
• un siège (2 ; 2') d'appui de la soupape (11) ménagé à l'intérieur du conteneur de façon que ladite soupape assure l'obturation du récipient (1), dans une position dite de fermeture,
• des moyens d'étanchéité (6, 13) aptes à assurer une étanchéité entre la soupape (11) et son siège d'appui (2 ; 2') dans la position de fermeture de ladite soupape,
• des moyens élastiques (29) disposés entre la paroi de stérilisation (7 ; 7') et la soupape (11) de façon à solliciter ladite soupape vers sa position de fermeture du récipient (1),
• et des moyens d'accrochage (15, 16) réalisés d'un seul tenant avec la soupape (11) et agencés pour s'étendre à l'extérieur du conteneur au travers d'un orifice (30) ménagé dans la paroi de stérilisation (7 ; 7'), lors d'un effort exercé sur ladite soupape à l'encontre des moyens élastiques (29), adapté pour amener cette dernière vers une position dite d'ouverture du récipient (1).
- les moyens d'enregistrement électronique comprennent un appareil électronique autonome (17) adapté pour être accolé à la paroi de stérilisation (7 ; 7') du conteneur, ledit appareil électronique :
- comprenant des moyens de verrouillage (19-24) adaptés pour coopérer avec les organes d'accrochage (15, 16) de la soupape (11), de façon à maintenir cette dernière dans sa position d'ouverture,
- comprenant des moyens (25) de déclenchement automatique des moyens de verrouillage (19-24) aptes à engendrer la libération des organes d'accrochage (15, 16) de la soupape (11) et à autoriser le déplacement de cette dernière vers sa position de fermeture sous l'action des moyens élastiques (29),
- étant programmé pour activer les moyens de déclenchement (25) lorsque chaque paramètre de stérilisation est respecté.

2. - Ensemble de stérilisation selon la revendication 1, **caractérisé en ce que** :
- la paroi de stérilisation (7 ; 7') est constituée d'un couvercle, dit sur-courvercle, de forme adaptée pour coiffer le récipient (1) et doté de moyens (8) de solidarisation audit récipient,
- le siège (2 ; 2') d'appui de la soupape (11) est constitué d'un couvercle de forme adaptée pour recouvrir le récipient (1), et percé d'au moins un orifice (4 ; 31) de passage d'un agent stérilisateur, apte à être obturé par la dite soupape dans la position de fermeture de cette dernière,
- des moyens d'étanchéité (3) sont disposés entre le récipient (1) et le couvercle (2 ; 2') de façon à assurer l'étanchéité du conteneur dans la position de fermeture de la soupape (11).

3. - Ensemble de stérilisation selon la revendication 2, **caractérisé en ce que** le couvercle (2') et le sur-couvercle (7') sont réalisés d'un seul tenant de façon à former un ensemble séparable du récipient (1) et incorporant la soupape (11).

4. - Ensemble de stérilisation selon la revendication 2, **caractérisé en ce que** le couvercle (2) et le sur-couvercle (7) consistent en deux éléments distincts, la soupape (11) étant disposée sous le sur-couvercle (7) et reliée à ce dernier par les moyens élastiques (29).

5. - Ensemble de stérilisation selon l'une des revendications 2 à 4, **caractérisé en ce que** le couvercle (2) est percé d'un orifice central (5), la soupape (11) étant constituée d'une plaque dotée en sous-face d'un doigt de guidage (14) apte à coulisser dans ledit orifice.

6. - Ensemble de stérilisation selon l'une des revendications 2 à 5, **caractérisé en ce que** le couvercle (2) comporte une portion centrale percée d'une pluralité d'orifices (4) de passage d'un agent stérilisateur, la soupape (11) étant constituée d'une plaque adaptée pour recouvrir ladite portion centrale, et lesdits couvercle et soupape comportant des moyens d'étanchéité (6, 13) ménagés de façon à être situés sur le pourtour de cette portion centrale.

7. - Ensemble de stérilisation selon l'une des revendications 2 à 6, **caractérisé en ce que** le sur-couvercle (7 ; 7') comporte une zone centrale (10) en renfoncement de dimensions adaptées pour loger l'appareil électronique (17).

8. - Ensemble de stérilisation selon l'une des revendications 2 à 7, **caractérisé en ce que** le sur-couvercle (7 ; 7') est percé d'au moins un orifice (9) de passage d'un agent stérilisateur.

9. - Ensemble de stérilisation selon l'une des revendications précédentes, **caractérisé en ce que** les moyens d'accrochage (15, 16) de la soupape (11), et les moyens élastiques (29) sollicitant ladite soupape vers sa position de fermeture du récipient (1), sont adaptés pour que lesdits moyens d'accrochage ne fassent pas saillie à l'extérieur de la paroi de stérilisation (7 ; 7'), dans la position de fermeture de la soupape (11).

10. - Ensemble de stérilisation selon l'une des revendications précédentes, **caractérisé en ce que** :
- les moyens d'accrochage (15, 16) de la soupape (11) et les moyens de verrouillage (19-24) de l'appareil électronique (17) comportent deux couronnes (15, 19) de formes adaptées pour venir s'emboîter l'une dans l'autre, et dotées d'organes (16, 20) de blocage relatif en translation axiale, une desdites couronnes étant composée de doigts (15) flexibles radialement,
- les moyens de verrouillage (19-24) de l'appareil électronique (17) comportent un verrou (21) disposé de façon à être repoussé par les organes d'accrochage (15, 16) de la soupape (11) lors du déplacement de cette dernière vers sa position d'ouverture, et sollicité par des moyens élastiques (24) aptes à le maintenir dans une position de butée radiale des doigts flexibles (15).
- les moyens de déclenchement automatique (25) sont adaptés pour entraîner un déplacement du verrou (21), à l'encontre de l'action des moyens élastiques (24), vers une position où il libère les doigts flexibles (15).

11. - Ensemble de stérilisation selon la revendication 10, **caractérisé en ce que** :
- les moyens d'accrochage (15, 16) de la soupape (11) consistent en la couronne composée de doigts flexibles (15), ladite couronne étant de forme adaptée pour venir coulisser à l'extérieur de la couronne (19) des moyens de verrouillage (19-24) de l'appareil électronique (17),
- le verrou (21) de l'appareil électronique (17) consiste en une pièce annulaire de forme adaptée pour venir enserrer les doigts flexibles (15).

12. - Ensemble de stérilisation selon la revendication 11, **caractérisé en ce que** la couronne (19) des moyens de verrouillage (19-24) de l'appareil électronique (17) comporte un tronçon d'extrémité (19a) présentant une face externe conique formant une rampe d'insertion à l'intérieur de la couronne (15) de la soupape (11).

13. - Ensemble de stérilisation selon l'une des revendications 10 à 12, **caractérisé en ce que** les organes (16, 20) de blocage en translation des couronnes (15, 19) consistent en un bossage annulaire (16) et une gorge annulaire (20) de formes conjuguées, l'organe de blocage (16) ménagé sur les doigts flexibles (15) étant situé au niveau de l'extrémité de ces derniers.

14. - Ensemble de stérilisation selon l'une des revendications 10 à 13, **caractérisé en ce que** le verrou (21) est réalisé en un matériau magnétique, les moyens de déclenchement dudit verrou consistant en un électro-aimant (25).

## Claims

1. Sterilisation set, in particular for medical instruments or appliances, having a container comprising a receptacle (1) and means (2, 7, 11 ; 2, 7', 11) for obturating said receptacle which are capable of obturating it in a sealed manner, and electronic means (17) for recording at least one sterilisation parameter during the sterilisation phases, said sterilisation set being
**characterized in that**:
- the container comprises:
• a valve (11) arranged inside the container opposite a wall (7 ; 7'), called a sterilisation wall, of said container, said wall being arranged to permit a sterilising agent to penetrate into said container,
• a seat (2 ; 2') for supporting the valve (11), said seat being formed inside the container so that said valve ensures the obturation of the receptacle (1), in a closed position,
• sealing means (6, 13) capable of ensuring a seal between the valve (11) and its supporting seat (2 ; 2') in the closed position of said valve,
• elastic means (29) arranged between the sterilisation wall (7 ; 7') and the valve (11) so as to stress said valve towards its position of closing the receptacle (1),
• and catching means (15, 16) made in one piece with the valve (11) and arranged to extend outside the container through an orifice (30) formed in the sterilisation wall (7 ; 7'), when a force is exerted on said valve countering the elastic means (29), designed to bring said valve towards a position of opening the receptacle (1).
- the electronic recording means comprise an independent electronic apparatus (17) designed to be attached to the sterilisation wall (7 ; 7') of the container, said electronic apparatus:
- comprising locking means (19-24) designed to cooperate with the catching members (15, 16) of the valve (11), so as to keep the latter in its open position,
- comprising means (25) for automatically disengaging the locking means (19-24) for producing the release of the catching members (15, 16) of the valve (11) and allowing the movement of the latter towards its closed position under the action of the elastic means (29),
- being programmed to activate the disengaging means (25) when every sterilisation parameter is met.

2. Sterilisation set according to claim 1,
**characterized in that**:
- the sterilisation wall (7 ; 7') consists of a lid, called an over-lid, of a shape designed to cap the receptacle (1) and equipped with means (8) for securing to said receptacle,
- the seat (2 ; 2') for supporting the valve (11) consists of a lid of a shape designed to cover the receptacle (1), and containing at least one orifice (4; 31) for the passage of a sterilising agent, which can be obturated by said valve in the closed position of the latter,
- sealing means (3) are arranged between the receptacle (1) and the lid (2 ; 2') so as to ensure the sealing of the container in the closed position of the valve (11).

3. Sterilisation set according to claim 2, **characterized in that** the lid (2') and the over-lid (7') are made in one piece so as to form an assembly separable from the receptacle (1) and incorporating the valve (11).

4. Sterilisation set according to claim 2, **characterized in that** the lid (2) and the over-lid (7) consist of two distinct elements, the valve (11) being arranged under the over-lid (7) and connected to the latter by the elastic means (29).

5. Sterilisation set according to one of claims 2 to 4, **characterized in that** the lid (2) contains a central orifice (5), the valve (11) consisting of a plate equipped at the lower face with a guiding pin (14) capable of sliding in said orifice.

6. Sterilisation set according to one of claims 2 to 5, **characterized in that** the lid (2) has a central portion containing a plurality of orifices (4) for the passage of a sterilising agent, the valve (11) consisting of a plate designed to cover said central portion, and said lid and valve having sealing means (6, 13) formed so as to be situated on the circumference of this central portion.

7. Sterilisation set according to one of claims 2 to 6, **characterized in that** the over-lid (7 ; 7') has a central indented zone (10) of dimensions designed to accommodate the electronic apparatus (17).

8. Sterilisation set according to one of claims 2 to 7, **characterized in that** the over-lid (7 ; 7') contains at least one orifice (9) for the passage of a sterilising agent.

9. Sterilisation set according to one of the preceding claims, **characterized in that** the catching means (15, 16) of the valve (11), and the elastic means (29) stressing said valve towards its position of closing the receptacle (1), are designed so that said catching means do not project outside the sterilisation wall (7 ; 7') in the closed position of the valve (11).

10. Sterilisation set according to one of the preceding claims, **characterized in that**:
- the catching means (15, 16) of the valve (11) and the locking means (19-24) of the electronic apparatus (17) have two rings (15, 19) of shapes designed to fit into each other, and equipped with members (16, 20) for relative blocking in terms of axial translation, one of said rings being composed of radially flexible fingers (15),
- the locking means (19-24) of the electronic apparatus (17) have a locking member (21) arranged so as to be pushed back by the catching members (15, 16) of the valve (11) during the movement of the latter towards its open position, and stressed by elastic means (24) capable of keeping it in a position of radially stopping the flexible fingers (15),
- the automatic disengaging means (25) are designed to bring about a movement of the locking member (21), countering the action of the elastic means (24), towards a position in which it releases the flexible fingers (15).

11. Sterilisation set according to claim 10,
**characterized in that**:
- the catching means (15, 16) of the valve (11) consist of the ring composed of flexible fingers (15), said ring being of a shape designed to slide on the outside of the ring (19) of the locking means (19-24) of the electronic apparatus (17),
- the locking member (21) of the electronic apparatus (17) consists of an annular part of a shape designed to enclose the flexible fingers (15).

12. Sterilisation set according to claim 11, **characterized in that** the ring (19) of the locking means (19-24) of the electronic apparatus (17) has an end section (19a) possessing a conical outer face forming a ramp for insertion inside the ring (15) of the valve (11).

13. Sterilisation set according to one of claims 10 to 12, **characterized in that** the members (16, 20) for blocking the rings (15, 19) in terms of translation consist of an annular boss (16) and an annular groove (20) of mating shapes, the blocking member (16) formed on the flexible fingers (15) being situated at the end of the latter.

14. Sterilisation set according to one of claims 10 to 13, **characterized in that** the locking member (21) is made from a magnetic material, the means for disengaging said locking member consisting of an electromagnet (25).

## Patentansprüche

1. Anlage für die Sterilisierung insbesondere von medizinischen Instrumenten oder Apparaten, die einen Container umfaßt, der einen Behälter (1) und Mittel zum Verschluß (2, 7, 11; 2, 7', 11) des besagten Behälters umfaßt, die geeignet sind, ihn dicht zu verschließen, und elektronische Mittel (17) zur Aufzeichnung von mindestens einem Parameter der Sterilisierung während der Sterilisierungsphasen, wobei die besagte Anlage für die Sterilisierung **dadurch gekennzeichnet ist, daß**:
- der Container folgende Elemente umfaßt:
. Ein Ventil (11), das im Innern des Containers vor einer Wand (7; 7'), der sogenannten Sterilisierungswand, des besagten Containers angeordnet ist, die derart gestaltet ist, daß das Eindringen eines Sterilisierungsmittels in letzteren möglich ist;
. Einen Auflagesitz (2; 2') des Ventils (11), der so im Innern des Containers gebildet ist, daß das besagte Ventil den Verschluß des Behälters (1) in einer sogenannten Verschlußposition sicherstellt;
. Dichtheitsmittel (6, 13), die geeignet sind, eine Abdichtung zwischen dem Ventil (11) und seinem Auflagesitz (2; 2') in der Verschlußposition des besagten Ventils zu sichern;
. Elastische Mittel (29), die zwischen der Sterilisierungswand (7; 7') und dem Ventil (11) angebracht sind, damit das besagte Ventil zu seiner Verschlußposition des Behälters (1) geführt wird;
. Anhängemittel (15, 16), die aus einem Stück mit dem Ventil (11) ausgeführt und so gestaltet sind, daß sie sich außerhalb des Containers durch eine in der Sterilisierungswand (7; 7') gebildete Öffnung (30) erstrecken, wenn eine Kraft auf das besagte Ventil gegen die elastischen Mittel (29) ausgeübt wird, die geeignet ist, letztere zu einer sogenannten Öffnungsposition des Behälters (1) zu führen.
- Die Mittel für die elektronische Aufzeichnung umfassen einen autonomen elektronischen Apparat (17), der geeignet ist, an der Sterilisierungswand (7; 7') des Containers befestigt zu werden, wobei der besagte elektronische Apparat folgende Teile umfaßt:
- Mittel zur Verriegelung (19 - 24), die geeignet sind, mit den Anhängeorganen (15, 16) des Ventils (11) so zusammenzuwirken, daß letzteres in seiner Öffnungsposition gehalten wird;
- Mittel (25) zum automatischen Auslösen der Mittel zur Verriegelung (19 - 24), die geeignet sind, die Freigabe der Anhängemittel (15, 16) des Ventils (11) auszulösen und die Verschiebung des letzteren zu ihrer Verschlußposition unter der Wirkung der elastischen Mittel (29) zuzulassen;
- Und so programmiert ist, daß die Auslösemittel (25) aktiviert werden, wenn jeder Parameter der Sterilisierung erfüllt wird.

2. Anlage für die Sterilisierung nach Anspruch 1,
**dadurch gekennzeichnet, daß**:
- Die Sterilisierungswand (7; 7') aus einem Deckel, einem sogenannten Überdeckel, in geeigneter Form gebildet wird, damit er den Behälter (1) überdeckt, und mit Mitteln (8) zur Solidarisierung am besagten Behälter ausgestattet ist;
- Der Auflagesitz 2; 2') des Ventils (11) aus einem Deckel in geeigneter Form gebildet wird, damit er den Behälter (1) aufnehmen kann, und mit mindestens einer gebohrten Öffnung (4; 31) für den Durchgang eines Sterilisierungsmittels, die geeignet ist, durch das besagte Ventil in der Verschlußposition des letzteren verschlossen zu werden;
- Dichtheitsmittel (3) zwischen dem Behälter (1) und dem Deckel (2; 2') so angeordnet sind, daß die Dichtheit des Containers in der Verschlußposition des Ventils (11) gesichert ist.

3. Anlage für die Sterilisierung nach Anspruch 2, **dadurch gekennzeichnet, daß** der Deckel (2') und der Überdeckel (7') so aus einem Stück realisiert sind, daß sie eine trennbare Einheit des Behälters (1) bilden und das Ventil (11) integrieren.

4. Anlage für die Sterilisierung nach Anspruch 2, **dadurch gekennzeichnet, daß** der Deckel (2) und der Überdeckel (7) aus zwei getrennten Elementen bestehen, wobei das Ventil (11) auf dem Überdeckel (7) angeordnet und mit den elastischen Mitteln (29) mit letzterem verbunden ist.

5. Anlage für die Sterilisierung nach einem beliebigen der vorstehenden Ansprüche 2 bis 4, **dadurch gekennzeichnet, daß** der Deckel (2) mit einer mittleren Öffnung (5) durchbohrt ist, wobei das Ventil (11) aus einer Platte besteht, die an ihrer Unterseite mit einem Führungszapfen (14) ausgestattet ist, der in der besagten Öffnung gleiten kann.

6. Anlage für die Sterilisierung nach einem beliebigen der vorstehenden Ansprüche 2 bis 5, **dadurch gekennzeichnet, daß** der Deckel (2) einen mit einer Vielzahl von Öffnungen (4) durchbohrten mittleren Abschnitt für den Durchgang eines Sterilisierungsmittels umfaßt, wobei das Ventil aus einer Platte besteht, die geeignet ist, den besagten mittleren Abschnitt abzudecken, und wobei der besagte Deckel und das besagte Ventil Dichtheitsmittel (6, 13) umfassen, die so gebildet sind, daß sie sich auf dem Umkreis dieses mittleren Abschnitts befinden.

7. Anlage für die Sterilisierung nach einem beliebigen der vorstehenden Ansprüche 2 bis 6, **dadurch gekennzeichnet, daß** der Überdeckel (7; 7') einen mittleren Bereich (10) zur Verstärkung mit Abmessungen umfaßt, die geeignet sind, den elektronischen Apparat (17) aufzunehmen.

8. Anlage für die Sterilisierung nach einem beliebigen der vorstehenden Ansprüche 2 bis 7, **dadurch gekennzeichnet, daß** der Überdeckel (7; 7') mit mindestens einer Öffnung (9) für den Durchgang eines Sterilisierungsmittels durchbohrt ist.

9. Anlage für die Sterilisierung nach einem beliebigen der vorstehenden Ansprüche, **dadurch gekennzeichnet, daß** die Anhängemittel (15, 16) des Ventils (11) und die elastischen Mittel (29), die das besagte Ventil zu seiner Verschlußposition des Behälters (1) führen, geeignet sind, damit die besagten Anhängemittel in der Verschlußposition des Ventils (11) keinen Vorsprung ab der Sterilisierungswand (7; 7') nach draußen bilden.

10. Anlage für die Sterilisierung nach einem beliebigen der vorstehenden Ansprüche, **dadurch gekennzeichnet, daß**:
- Die Anhängemittel (15, 16) des Ventils (11) und die Verriegelungsmittel (19 - 24) des elektronischen Apparats (17) zwei Kränze (15, 19) in Formen umfassen, die geeignet sind, ineinander zu greifen, und mit relativen Blockierorganen (16, 20) in axialer Translation versehen sind, wobei einer der besagten Kränze aus radial flexiblen Zapfen (15) besteht;
- Die Verriegelungsmittel (19 - 24) des elektronischen Apparats (17) einen Riegel (21) umfassen, der so angeordnet ist, daß er von den Anhängemitteln (15, 16) des Ventils (11) bei der Verschiebung des letzteren zu seiner Öffnungsposition zurück gedrückt wird und von elastischen Mitteln (24) belastet wird, die geeignet sind, ihn in einer radialen Anschlagposition der flexiblen Zapfen (15) zu halten;
- Die Mittel zur automatischen Auslösung (25) geeignet sind, eine Verschiebung des Riegels (21) gegen die Wirkung der elastischen Mittel (24) zu einer Position auszulösen, wo er die flexiblen Zapfen (15) freigibt.

11. Anlage für die Sterilisierung nach Anspruch 10, **dadurch gekennzeichnet, daß**:
- Die Anhängemittel (15, 16) des Ventils (11) aus dem aus flexiblen Zapfen (15) zusammengesetzten Kranz bestehen, wobei der besagte Kranz die geeignete Form hat, um außerhalb des Kranzes (19) der Verriegelungsmittel (19 - 24) des elektronischen Apparats (17) zu gleiten;
- Der Riegel (21) des elektronischen Apparats (17) aus einem ringförmigen Teil in geeigneter Form besteht, damit er die flexiblen Zapfen (15) umschließt.

12. Anlage für die Sterilisierung nach Anspruch 11, **dadurch gekennzeichnet, daß** der Kranz (19) der Verriegelungsmittel (19 - 24) des elektronischen Apparats (17) einen Endabschnitt (19a) umfaßt, der eine kegelförmige Außenseite aufweist, die eine Rampe zum Einsetzen ins Innere des Kranzes (15) des Ventils (11) umfaßt .

13. Anlage für die Sterilisierung nach einem beliebigen der vorstehenden Ansprüche 10 bis 12, **dadurch gekennzeichnet, daß** die Organe (16, 20) zum Blockieren in Translation der Kränze (15, 19) aus einem ringförmigen Höcker (16) und einer ringförmigen Kehle (20) in konjugierten Formen bestehen, wobei das auf den flexiblen Zapfen (15) gebildete Blockierorgan (16) sich am Ende der letzteren befindet.

14. Anlage für die Sterilisierung nach einem beliebigen der vorstehenden Ansprüche 10 bis 13, **dadurch gekennzeichnet, daß** der Riegel (21) aus einem magnetischen Material ausgeführt ist, wobei die Mittel zur Auslösung des besagten Riegels aus einem Elektromagneten (25) bestehen.
